# EUROPEAN PATENT SPECIFICATION

(11) **EP 1 885 727 B1**
(45) Date of publication and mention of the grant of the patent: **03.03.2010**
(21) Application number: 06809025.7
(22) Date of filing: 03.05.2006
(51) Int. Cl.: C07D 491/10

(54) **SYNTHESES AND PREPARATIONS OF NARWEDINE AND RELATED NOVEL COMPOUNDS**
SYNTHESEN UND HERSTELLUNGEN VON NARWEDIN UND VERWANDTEN NEUEN VERBINDUNGEN
SYNTHESES ET PREPARATIONS DE NARWEDINE ET DE NOUVEAUX COMPOSES ASSOCIES

(30) Priority: 03.05.2005 US 676964 P; 30.09.2005 US 722015 P
(43) Date of publication of application: 13.02.2008
(73) Proprietor: Medichem, S.A., 08970 Sant Joan Despi, Barcelona (ES)
(72) Inventor: TOJO SUAREZ, Gabriel, E-15895 Ames (A Coruna) (ES); DURAN LOPEZ, Ernesto, E-08904 Hospitalet De Llobregat (ES); BOSCH I LLADO, Jordi, E-17003 Girona (ES)
(74) Representative: HOFFMANN EITLE
(86) International application number: PCT/IB2006/002875
(87) International publication number: WO 2007/010412

(56) References cited:
- WO-A-96/31458
- WO-A2-2006/072818
- US-A- 6 043 359

## Description

### BACKGROUND OF THE INVENTION

### Field of the Invention

The invention relates to a process for preparing racemic narwedine (which can be can be kinetically resolved to yield (-)-narwedine and which is the biogenic precursor of (-)-galanthamine) and the use thereof as a starting material for producing (-)-galanthamine. The invention further includes processes for preparing (-)-galanthamine and (-)-galanthamine hydrobromide.

### Discussion of the Related Art

(-)-Galanthamine, a tertiary alkaloid having acetylcholinesterase inhibitor properties, has been isolated from the bulbs of the Caucasian snowdrops *Galanthus woronowii.* It has also been isolated from the common snowdrop *Galanthus nivalis.*
(-)-Galanthamine has been marketed by Waldheim (Sanochemia Gruppe) as Nivalin® in Germany and Austria since the 1970s for indications such as facial neuralgia.
(-)-Galanthamine, and derivatives thereof, are useful for the treatment of Alzheimer's disease and related illnesses.
(-)-Galanthamine hydrobromide has been approved by the FDA for the treatment of Alzheimer's disease.

The chemical name of (-)-galanthamine is (4aS,6R,8aS)-4a, 5, 9, 10, 11, 12-hexahydro-3-methoxy- 11-methyl-6H-benzofuro[3a, 3, 2-ef][2]benzazepin-6-ol. Both the base compound and its hydrobromide are levorotatory.

The crystal structure (-)-galanthamine hydrobromide is described in *Acta Cryst*., C53, 1284-1286 (1997).

Currently, (-)-galanthamine is obtained by extraction from natural sources, such as daffodils or snowdrops. The yields of these extraction process are low, expensive for pharmaceutical grade material and limited supplies of naturally-obtained (-)-galanthamine. (-)-Narwedine is the biogenic precursor of (-)-galanthamine.

An alternate source of (-)-galanthamine is its chemical synthesis. Some synthetic routes to (-)-galanthamine and racemic galanthamine have been published including, for example, (i) Barton and Kirby, J. Chem. Soc., 806 (1962); (ii) Shimizu et at, Heterocycles, 8, 277 (1977); (iii) Shimizu et at, Chem. Pharm. Bull., 26, 3765 (1978); (iv) Szewczyk et at, J. Heterocycl. Chem., 25, 1809 (1988); (v) Kametani et at, J. Org. Chem., 36, 1295 (1971); (vi) Kametani et al, J. Chem. Soc. Perkin Trans. 1, 1513 (1972); (vii) Kametani et al, J. Heterocycl. Chem., 10, 35 (1973); (viii) Vlahov et al, Tetrahedron, 45, 3329 (1989) and (ix) Holton et al, J. Am. Chem. Soc., 110, 314 (1988).

In J. Chem. Soc. (C), 2602-2605 (1969), the oxidative ring coupling of 2-bromo-5-hydroxy-N-[2-(4-hydroxyphenyl)ethyl]-4-methoxy-N-methylbenzamide (Compound 4) to 1-bromo-12-oxo-narwedine (Compound 5) was first described as a key step in the synthesis of racemic narwedine. Notably, the oxidation is described as being performed in a mixture of chloroform and water (5:1, 300 volumes) as solvent, using 5.66 molar equivalents of potassium hexacyanoferrate (III) as oxidant and 11.32 molar equivalents of sodium bicarbonate as base, at 60° C for 1.5 hours, with a 40% yield after purification by means of column chromatography.

WO 96/31458 describes the oxidative ring coupling of Compound 4 in a two-phase liquid system made up of an aqueous base and an organic solvent having a dielectric constant less than 4.8 (as measured at 20° C). Example 2 of WO 96/31458 is carried out with Compound 4 as starting material and a mixture of toluene and water (2:1, 150 volumes) as solvent, using 3.00 molar equivalents of potassium hexacyanoferrate (III) as oxidant and 11.32 molar equivalents of sodium bicarbonate as base, at reflux temperature (87° C) for 3 hours. The yield was 19.1%.

Example 34 of WO 96/12692 describes the oxidative ring coupling of Compound 4 in a mixture of toluene and water (15:1, 320 volumes) as solvent, using 5.20 molar equivalents of potassium hexacyanoferrate (III) as oxidant and 5.50 molar equivalents of potassium carbonate as base, at 60° C for 35 minutes, with a 37.8% yield after chromatographic purification. Potassium carbonate is a dibasic salt and therefore the reaction was carried out using 11.0 molar equivalents of base.

Synth. Commun., 30, 2833-2846 (2000) describes the oxidative ring coupling of Compound 4 using phenyliodine(III)bis(trifluoroacetate) (PIFA) as oxidant in trifluoroethanol at -40° C in a 60% yield after column chromatography. Notwithstanding the yield, the need for chromatographic purification, the high cost of both oxidant and solvent, and also the low temperatures needed make the process inadequate for an industrial production.

J. Chem. Soc. (C), 2602-2605 (1969) describes the reduction of 1-bromo-12-oxo-narwedine (Compound 5) using lithium aluminum hydride that yields a mixture of about 60:40 of galanthamine and epigalanthamine, where both are in their racemic forms. This mixture is then further purified by means of column chromatography to yield both products in their racemic forms.

J. Heterocyclic Chem., 32, 195-199 (1995) describes a method for resolving racemic galanthamine that involves derivatizing galanthamine using (-)-camphanic acid chloride and separating the diastereomeric mixture using column chromatography. The (+)-galanthamine present in the racemic mixture is of little or no commercial value and it can not be transformed into the desired enantiomer.

In this regard, U.S. Patent No. 6,043,359 describes the preparation of narwedine from Compound 5 above (see examples 35 and 36) in which the carbonyl of Compound 5 is protected by using propyleneglycol to form a ketal (Scheme 1). The yield of this protection step is low (∼30.1%). Thereafter, the amido group and bromoaryl functionality are reduced with LiAlH₄, and the carbonyl is deprotected to yield narwedine. The yield of these last two steps is 61.2%, and the overall yield of converting Compound 5 to narwedine is only 18.4%

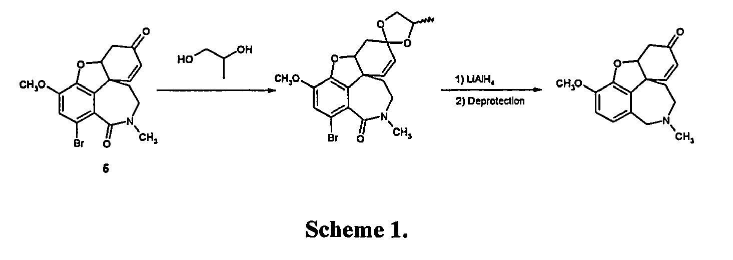

U.S. Patent No. 5,428,159 (the "'159 patent") describes the reduction of (-)-narwedine to (-)-galanthamine. According to Example VIII of the '159 patent, (-)-narwedine is reduced at -78° C with L-selectride in THF to (-)-galanthamine. The mixture solution is stirred at -78° C for 2 hours and then warmed to 0° C. Methanol is added, and the solution is warmed to 25° C and stirred for 15 minutes. Solvent is then evaporated under vacuum to dryness to obtain a syrup. The syrup is dissolved in chloroform and the solution is loaded into a SiO₂ column. The column is eluted with a solvent mixture of chloroform and methanol (6/1) to obtain 286 mg of pure (-)-galanthamine (a yield of 99.5%). HPLC of the resultant mixture indicates there is no epigalanthamine or lycoramine present (m.p. 128° C - 129° C; [α] _{D}²⁵ = -93.4). Natural (-)-galanthamine has a melting point of 126-127° C ([α] _{D}²⁵ = 91.0).

Reactions at -78° C, however, are not very suitable to large scale production. Furthermore, the desired product must be purified by column chromatography, which is not affordable at industrial scale.

Org. Process Res. Dev., 3, 425-431 (1999) discloses that solutions of (-)-narwedine in tetrahydrofuran undergo racemization, and because of that, solid (-)-narwedine is portionwise added through a solid-addition funnel to solutions of L-selectride at temperatures below -15° C. Additionally, Org. Process Res. Dev., 3, 425-431 (1999) discloses that the final (-)-galanthamine is isolated after formation of its hydrobromic acid salt and further filtration. Although the disclosed process indicates that the reduction reaction is carried out under an argon atmosphere, no information is provided regarding oxygen removal in subsequent steps and/or the purity of the final product.

Notwithstanding these advances, the synthetic approach to (-)-galanthamine production in high purity pharmaceutical grade on a commercial scale is still problematic.

The invention includes processes having reaction conditions that improve significantly the yield of the oxidative ring coupling of 2-bromo-5-hydroxy-N-[2-(4-hydroxyphenyl)ethyl]-4-methoxy-N-methylbenzamide to produce 1-bromo-12-oxo-narwedine, which is a key intermediate in some synthetic routes to racemic narwedine.

### BRIEF DESCRIPTION OF THE DRAWINGS

The accompanying drawings, which are included to provide a further understanding of the invention and are incorporated in and constitute a part of this specification, illustrate embodiments of the invention and together with the description serve to explain the principles of the invention. In the drawings:

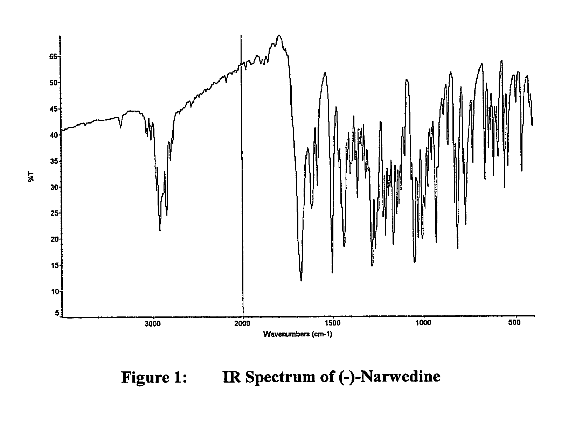

Figure 1 illustrates the IR spectrum of (-)-narwedine;

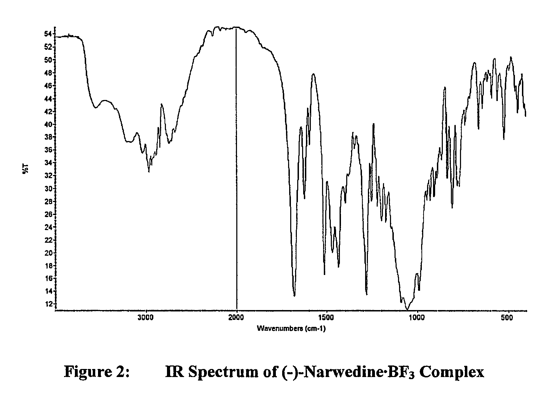

Figure 2 illustrates the IR spectrum of (-)-narwedine·BF₃ complex;

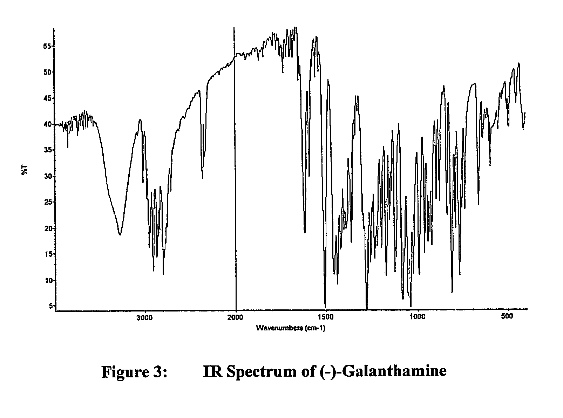

Figure 3 illustrates the IR spectrum of (-)-galanthamine;

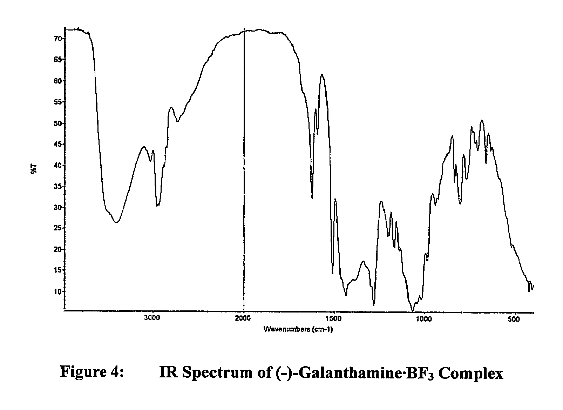

Figure 4 illustrates the IR spectrum of (-)-galanthamine·BF₃ complex;

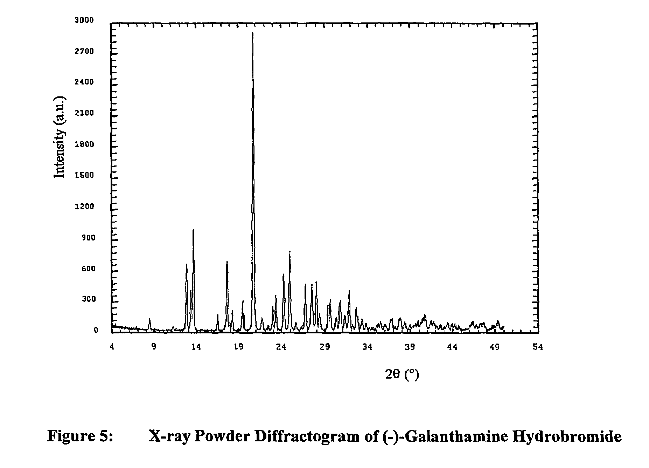

Figure 5 illustrates the X-ray powder diffractogram of (-)-galanthamine hydrobromide;

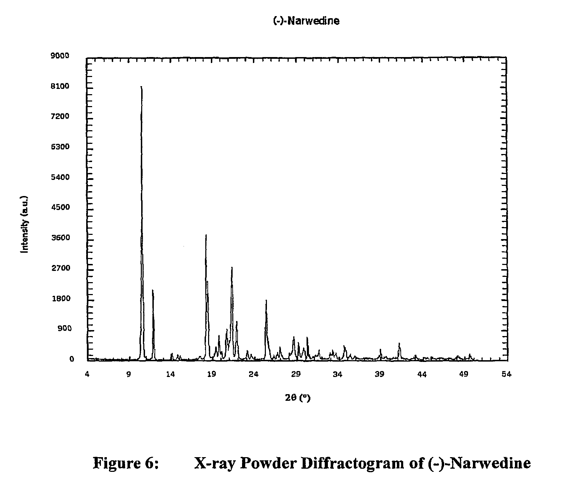

Figure 6 illustrates the X-ray powder diffractogram of (-)-narwedine; and

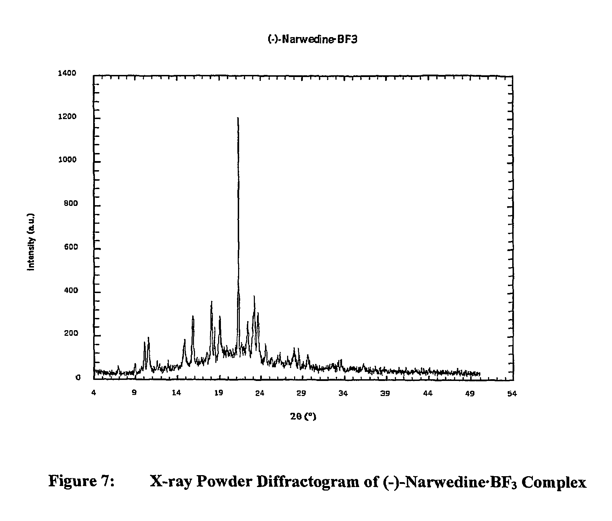

Figure 7 illustrates the X-ray powder diffractogram of (-)-narwedine·BF₃ complex.

### DETAILED DESCRIPTION OF THE PREFERRED EMBODIMENTS

Reference will now be made in detail to the preferred embodiments of the invention. This invention may, however, be embodied in many different forms and should not be construed as limited to the embodiments set forth herein. In addition, and as will be appreciated by one of skill in the art, the invention may be embodied as a method, system or process.

The invention relates to the use of a limited amount of base in the oxidative ring coupling of 2-bromo-5-hydroxy-*N*-[2-(4-hydroxyphenyl)ethyl]-4-methoxy-*N*-methylbenzamide (Compound 4) to 1-bromo-12-oxo-narwedine (Compound 5). This process provides a high yield in the transformation when, potassium hexacyanoferrate (III) is used as the oxidant and without the need of chromatographic purification, thus making the process suitable for an industrial production. The oxidant is used in conjunction with less than 10 molar equivalents of at least one base.

### Specific Examples

The following examples are for illustrative purposes only and are not intended, nor should they be interpreted to, limit the scope of the invention.

### General Experimental Conditions:

### i. BPLC Method 1:

Chromatographic separation was carried out using a Phenomenex Luna C18, 5 µm, 4.6 x 150 mm I.D column at room temperature (∼20-25° C).

The mobile phase A was 0.01 M ammonium bicarbonate buffer (pH=7.5) which was prepared from 0.79 g of NH₄HCO₃ dissolved in 1000 mL of water. The pH was adjusted to 7.5 with formic acid. The mobile phase was mixed and filtered through a 0.22 µm nylon filter under vacuum.

The mobile phase B was acetonitrile.

The chromatograph was programmed as follows: 0-20 minutes isocratic 85% mobile phase A, 20-25 minutes linear gradient to 75% mobile phase A, 25-45 minutes isocratic 75% mobile phase A, 45-47 minutes linear gradient to 85% mobile phase A and 47-60 minutes equilibration to 85% mobile phase A.

The chromatograph was equipped with a 232 nm detector, and the flow rate was 1.0 mL per minute. Test samples (10 µL) were prepared by dissolving the appropriate amount of sample in order to obtain 1.0 mg per mL of a 85:15 mixture of mobile phases A and B.

### ii. HPLC Method 2 (Chiral):

Chromatographic separation was carried out using a Daicel Chiralcel OD-H, 5 µm, 4.6 x 150 mm I.D column at room temperature (∼20-25° C).

The mobile phase A was hexane.

The mobile phase B was ethanol.

The chromatograph was programmed as follows: Initial 95% mobile phase A, 0-15 minutes linear gradient to 90% mobile phase A, 15-20 minutes isocratic 90% mobile phase A, 20-25 minutes linear gradient to 85% mobile phase A, 25-45 minutes isocratic 85% mobile phase A, 45-46 minutes linear gradient to 95% mobile phase A and 46-55 minutes equilibration to 95% mobile phase A.

The chromatograph was equipped with a 218 nm detector, and the flow rate was 1.0 mL per minute. Test samples (10 µL) were prepared by dissolving the appropriate amount of sample in order to obtain 1.0 mg per mL of a 50:50 mixture of hexane and sodium ethoxide solution (0.25 mg/ml) in ethanol.

### iii. HPLC Method 3

Chromatographic separation was carried out using a Phenomenex Luna C18, 5 µm, 4.6 x 150 mm I.D column at room temperature (∼20-25° C).

The mobile phase A was 0.01 M ammonium bicarbonate buffer (pH=7.5) which was prepared from 0.79 g of NH₄HCO₃ dissolved in 1000 mL of water. The pH was adjusted to 7.5 with formic acid. The mobile phase was mixed and filtered through a 0.22 µm nylon filter under vacuum.

The mobile phase B was acetonitrile.

The chromatograph was programmed as follows: Initial 0-20 minutes isocratic 85% mobile phase A, 20-25 minutes linear gradient to 75% mobile phase A, 25-45 minutes isocratic 75% mobile phase A, 45-47 minutes linear gradient to 85% mobile phase A and 47-60 minutes equilibration to 85% mobile phase A.

The chromatograph was equipped with a 232 nm detector, and the flow rate was 1.0 mL per minute. Test samples (10 µL) were prepared by dissolving the appropriate amount of sample in order to obtain 1.0 mg per mL of a 85:15 mixture of mobile phases A and B.

### iv. HPLC Method 4

Chromatographic separation was carried out using a Waters Symmetry C 18, 5 µm, 4.6 x 150 mm I.D column at room temperature (∼20-25° C).

The mobile phase A was a mixture of 0.065 M ammonium acetate buffer (pH=6.5), which was prepared from 5.01 g of CH₃COONH₄ dissolved in 1000 ml of water. The pH was adjusted to 6.5 with acetic acid, and acetonitrile (65:35, v/v). The mobile phase was mixed and filtered through a 0.22 µm nylon filter under vacuum.

The mobile phase B was acetonitrile.

The chromatograph was programmed as follows: Initial 0-15 minutes isocratic 100% mobile phase A, 15-20 minutes linear gradient to 45% mobile phase A, 20-50 minutes isocratic 45% mobile phase A, 50-55 minutes linear gradient to 100% mobile phase A and 55-70 minutes equilibration to 100% mobile phase A.

The chromatograph was equipped with a 230 nm detector, and the flow rate was 1.0 mL per minute. Test samples (10 µL) were prepared by dissolving the appropriate amount of sample in order to obtain 1.0 mg per mL of mobile phase A for 2-bromo-5-hydroxy-*N-*[2-(4-hydroxyphenyl)ethyl]-4-methoxy-*N*-methylbenzamide or mobile phase B for 5-benzyloxy-*N*-[2-(4-benzyloxyphenyl)ethyl]-2-bromo-4-methoxy-*N*-methylbenzamide.

### v. HPLC Method 5

Chromatographic separation was carried out using a Waters Symmetry C 18, 5 µm, 4.6 x 150 mm I.D column at room temperature (∼20-25° C).

The mobile phase A was water.

The mobile phase B was acetonitrile.

The chromatograph was programmed as follows: Initial 0-10 minutes isocratic 70% mobile phase A, 10-18 minutes linear gradient to 30% mobile phase A, 18-30 minutes isocratic 30% mobile phase A, 30-35 minutes linear gradient to 70% mobile phase A and 35-40 minutes equilibration to 70% mobile phase A.

The chromatograph was equipped with a 225 nm detector, and the flow rate was 1.0 mL per minute. Test samples (10 µL) were prepared by dissolving the appropriate amount of sample in order to obtain 1.0 mg per mL of acetonitrile.

### vi. HPLC Method 6

Chromatographic separation was carried out using a Phenomenex Luna C18, 5 µm, 4.6 x 150 mm I.D column at 25° C.

The mobile phase was 0.01 M ammonium bicarbonate buffer (pH=7.5) which was prepared from 0.79 g of NH₄HCO₃ dissolved in 1000 mL of water. The pH was adjusted to 7.5 with formic acid and acetonitrile (85:15, v/v). The mobile phase was mixed and filtered through a 0.22 µm nylon filter under vacuum.

The chromatograph was equipped with a 232 nm detector, and the flow rate was 1.5 mL per minute. Test samples (10 µL) were prepared by dissolving the appropriate amount of sample in order to obtain 1.0 mg per mL of mobile phase.

### vii. HPLC Method 7

Chromatographic separation was carried out using a Phenomenex Luna C18, 5 µm, 4.6 x 150 mm I.D column at room temperature (∼20-25° C).

The mobile phase A was 0.01 M ammonium bicarbonate buffer (pH=7.5) which was prepared from 0.79 g of NE₄HCO₃ dissolved in 1000 mL of water. The pH was adjusted to 7.5 with formic acid. The mobile phase was mixed and filtered through a 0.22 µm nylon filter under vacuum.

The mobile phase B was acetonitrile.

The chromatograph was programmed as follows: Initial 0-20 minutes isocratic 85% mobile phase A, 20-25 minutes linear gradient to 75% mobile phase A, 25-65 minutes isocratic 75% mobile phase A, 65-67 minutes linear gradient to 85% mobile phase A and 67-80 minutes equilibration to 85% mobile phase A.

The chromatograph was equipped with a 232 nm detector, and the flow rate was 1.0 mL per minute. Test samples (10 µL) were prepared by dissolving the appropriate amount of sample in order to obtain 1.0 mg per mL of a 85:15 mixture of mobile phases A and B.

### viii. HPLC Method 8 (Chiral)

Chromatographic separation was carried out using a Daicel Chiralcel OD-H, 5 µm, 4.6 x 150 mm I.D. column at room temperature (∼20-25° C).

The mobile phase was hexane/ethanol (1% acetic acid) (50:50, v/v).

The chromatograph was equipped with a 230 nm detector, and the flow rate was 1.0 mL per minute. Test samples (40 µL) were freshly prepared by dissolving the appropriate amount of sample in order to obtain 0.25 mg per ml of mobile phase.

### Example 1: Synthesis of 5-Benzyloxy-N-[2-(4-benzyloxyphenyl)ethyl]-2-brom bromo-4-methoxy-N-methylbenzamide (Compound 3)

A solution of 48.7 g of 5-benzyloxy-2-bromo-4-methoxybenzoic acid (Compound 2) in 1.08 L of toluene was heated to reflux, and residual water was removed azeotropically. The solution was cooled to 68° C, and 4.8 mL of *N*,*N*-dimethylformamide was added to the resulting suspension, followed by the addition of 12.6 mL of thionyl chloride. The suspension was then stirred at this temperature for two hours. Next, 350 mL of toluene was distilled at reflux temperature to remove excess of thionyl chloride, and the solution was cooled to room temperature. At this temperature, 24 mL of triethylamine was added to the solution. Thereafter, an anhydrous solution of 34.7 g of 2-(4-benzyloxyphenyl)ethylmethylamine (Compound 1) in 140 mL of toluene was added to the reaction mixture over 30 minutes. The resulting solution was stirred at room temperature for 1 hour. The solution was then washed with 200 mL of 5% NaHCO₃ solution, followed by 200 mL of 0.1 M HCl solution, and finally with 200 mL of water.

The organic phase was extracted and concentrated to a final volume of 160 mL. Next, 240 mL of isopropanol was added to the solution, and 80 mL of the resulting mixture of solvents was distilled at atmospheric pressure. After cooling to 40 °C, the resulting solution was seeded with 5-benzyloxy-*N*-[2-(4-benzyloxyphenyl)ethyl]-2-bromo-4-methoxy-*N-*methylbenzamide (Compound 3). Next, the suspension was cooled at room temperature overnight and finally at 0 °C for 90 minutes. A pale beige solid was filtered using a Büchner funnel and washed with 160 mL of chilled isopropanol to yield 60.3 g of 5-benzyloxy-*N*-[2-(4-benzyloxyphenyl)ethyl]-2-bromo-4-methoxy-*N*-methylbenzamide (Compound 3) (Yield: 74.7%; HPLC (method 4) purity 94.5%).

### Example 2: Synthesis of 2-Bromo-5-hydroxy-N-[2-(4-hydroxyphenyl)ethyl]-4-methoxy-N-methylbenzamide (Compound 4)

5-Benzyloxy-*N*-[2-(4-benzyloxyphenyl)ethyl]-2-bromo-4-methoxy-*N*-methylbenzamide (5.0 g) (Compound 3) was dissolved in a mixture of toluene (50 mL) and 48% hydrobromic acid (71 mL). The resulting solution was stirred at 60° C for 2 hours. After cooling to room temperature, the aqueous phase was extracted. CH₂Cl₂ (25 mL) was added over the aqueous phase, and the resulting mixture was cooled to 0° C. At this temperature, 120 mL of 20% aqueous NaOH solution was slowly added while maintaining the reaction temperature below 5 °C. Precipitation of a white solid was observed during the addition. The pH was adjusted to 5 with NaHCO₃, and the solid was filtered using a Büchner funnel and washed with 6 mL of water and 6 mL of heptane to obtain a quantitative yield 2-Bromo-5-hydroxy-*N*-[2-(4-hydroxyphenyl)ethyl]-4-methoxy-*N*-methylbenzamide (Compound 4) (HPLC (method 4) purity 95.9%).

### Example 3: Synthesis of 1-bromo-12-oxo-Narwedine (Compound 5) Using Dichloromethane as Solvent

In a 1L, three necked round bottom flask equipped with mechanical stirring and N₂ inlet, 2.0 g (5.3 mmol) of 2-bromo-5-hydroxy-*N*-[2-(4-hydroxyphenyl)ethyl]-4-methoxy-*N-*methylbenzamide (Compound 4) was dissolved in 600mL of CH₂Cl₂. To the resulting solution H₂O (40 mL), KHCO₃ (2.9 g, 29 mmol) and K₃[Fe(CN)₆] (8.4 g, 26 mmol) were added, and the resulting mixture was stirred at room temperature (∼20-25° C) for 24 hours. The resulting mixture was filtered using a Büchner funnel and the mother liquors were decanted. The organic layer was evaporated to dryness to yield 1.4 g of 1-bromo-12-oxo-narwedine (Compound 5) (Yield: 71%; HPLC (method 5) purity 87%).

### Example 4: Synthesis of 1-Bromo-12-oxo-Narwedine (Compound 5) using Toluene as Solvent

In a 1L, three necked round bottom flask equipped with mechanical stirring and N₂ inlet, 2.0 g (5.3 mmol) of 2-bromo-5-hydroxy-*N*-[2-(4-hydroxyphenyl)ethyl]-4-methoxy-*N-*methylbenzamide (Compound 4) was dissolved in 600mL of toluene. To the resulting solution H₂O (40 mL), KHCO₃ (2.9 g, 29 mmol) and K₃[Fe(CN)₆] (8.4 g, 26 mmol) were added, and the resulting mixture was stirred at room temperature (∼20-22° C) for 20 hours. The resulting mixture was filtered using a Büchner funnel and the mother liquors were decanted. The organic layer was evaporated to dryness to yield 1.22 g of 1-bromo-12-oxo-narwedine (Compound 5) (Yield: 61%; HPLC (method 5) purity 90%).

### Examples 5-12

Examples 5-12 illustrate the reaction results obtained when Example 4 is performed using different solvents.

| **Example** | **Solvent** | **Base** | **Yield** | **HPLC purity** (method 5) |
|---|---|---|---|---|
| 5 | AcO^{t}Bu | KHCO₃ (5.5 eq) | 91 % | 71 % |
| 6 | AcO^{t}Pr | KHCO₃ (5.5 eq) | 91 % | 35 % |
| 7 | AcOEt | KHCO₃ (5.5 eq) | 67 % | 79 % |
| 8 | AcOⁿBu | KHCO₃ (5.5 eq) | 54 % | 28 % |
| 9 | Xylene | KHCO₃ (5.5 eq) | 16 % | 94 % |
| 10 | Methyl *tert*-butyl ether | KHCO₃ (5.5 eq) | 8 % | 81 % |
| 11 | Chloroform | KHCO₃ (5.5 eq) | 52 % | 70 % |
| 12 | THF | KHCO₃ (5.5 eq) | 19 % | 15 % |

### Examples 13-23

Examples13-23 illustrate the reaction results obtained when Example 4 is performed using a different base or a different amount of base.

| **Example** | **Solvent** | **Base** | **Yield** | **HPLC purity** (method 5) |
|---|---|---|---|---|
| 13 | Toluene | KHCO₃ (5.5 eq) | 61 % | 90 % |
| 14 | Toluene | KHCO₃ (2.0 eq) | 28 % | 67 % |
| 15 | Toluene | KHCO₃ (1.1 eq) | 21 % | 47 % |
| 16 | Toluene | --- | 0 % | --- |
| 17 | Toluene | KHCO₃ (11.0 eq) | 24 % | 89 % |
| 18 | Toluene | KHCO₃/LiCl (5.5 eq) | 37 % | 78 % |
| 19 | Toluene | Li₂CO₃ (5.5 eq) | 38 % | 80 % |
| 20 | Toluene | NEt₃ (5.5 eq) | 43 % | 87 % |
| 21 | Toluene | Diisopropylethylamine (5.5 eq) | Not isolated. Detected by TLC | --- |
| 22 | Toluene | Pyridine (5.5 eq) | 0 % | --- |
| 23 | Toluene | NH₃ (5.5 eq) | Not isolated. Detected by TLC | --- |

## Claims

1. A process for reacting 2-bromo-5-hydroxy-N-[2-(4-hydroxyphenyl)ethyl]-4-methoxy-N-methylbenzamide (Compound 4) to obtain 1-bromo-12-oxo-narwedine (Compound 5) comprising treating Compound 4 with potassium hexacyanoferrate (III) as an oxidant in conjunction with less than 10 molar equivalents of at least one base.

2. The process of claim 1, wherein the at least one base comprises less than 8 molar equivalents.

3. The process of claim 2, wherein the at least one base comprises 5 molar equivalents.

4. The process of claim 1, 2 or 3, wherein the at least one base is at least one of an inorganic base, an organic base, NH₃ and combinations thereof.

5. The process of claim 4, wherein the base is potassium bicarbonate.

6. The process of any one of claims 1 to 5, wherein said reaction of said Compound 4 is performed at a temperature between 0°C and 50°C.

7. The process of claim 6, wherein the temperature is between 15°C and 35°C.

8. The process of claim 6, wherein the temperature is between 20°C and 25°C.

## Patentansprüche

1. Verfahren zur Umsetzung von 2-Brom-5-hydroxy-N-[2-(4-hydroxyphenyl)ethyl]-4-methoxy-N-methylbenzamid (Verbindung 4), um 1-Brom-12-oxo-narwedin (Verbindung 5) zu erhalten, umfassend die Behandlung von Verbindung 4 mit Kaliumhexacyanoferrat(III) als ein Oxidationsmittel in Verbindung mit weniger als 10 Moläquivalenten mindestens einer Base.

2. Verfahren gemäss Anspruch 1, wobei die mindestens eine Base weniger als 8 Moläquivalente umfasst.

3. Verfahren gemäss Anspruch 2, wobei die mindestens eine Base 5 Moläquivalente umfasst.

4. Verfahren gemäss Anspruch 1, 2 oder 3, wobei die mindestens eine Base mindestens eine aus einer anorganischen Base, einer organischen Base, NH₃ und Kombinationen davon ist.

5. Verfahren gemäss Anspruch 4, wobei die Base Kaliumbicarbonat ist.

6. Verfahren gemäss einem der Ansprüche 1 bis 5, wobei die Umsetzung von Verbindung 4 bei einer Temperatur zwischen 0 und 50°C durchgeführt wird.

7. Verfahren gemäss Anspruch 6, wobei die Temperatur zwischen 15 und 35°C liegt.

8. Verfahren gemäss Anspruch 6, wobei die Temperatur zwischen 20 und 25°C liegt.

## Revendications

1. Procédé pour faire réagir du 2-bromo-5-hydroxy-N-[2-(4-hydroxyphényl)éthyl]-4-méthoxy-N-méthylbenzamide (composé 4) pour obtenir du 1-bromo-12-oxo-narwédine (composé 5) comprenant le traitement du composé 4 avec de l'hexacyanoferrate de potassium (III) comme oxydant conjointement à moins de 10 équivalents molaires d'au moins une base.

2. Procédé selon la revendication 1, dans lequel la au moins une base comprend moins de 8 équivalents molaires.

3. Procédé selon la revendication 2, dans lequel la au moins une base comprend 5 équivalents molaires.

4. Procédé selon la revendication 1, 2 ou 3, dans lequel la au moins une base est au moins une parmi une base inorganique, une base organique, NH₃ et les combinaisons de celles-ci.

5. Procédé selon la revendication 4, dans lequel la base est le bicarbonate de potassium.

6. Procédé selon l'une quelconque des revendications 1 à 5, dans lequel ladite réaction dudit composé 4 est réalisée à une température entre 0°C et 50°C.

7. Procédé selon la revendication 6, dans lequel la température est entre 15°C et 35°C.

8. Procédé selon la revendication 6, dans lequel la température est entre 20°C et 25°C.
